# EUROPEAN PATENT SPECIFICATION

(11) **EP 0 408 547 B1**
(45) Date of publication and mention of the grant of the patent: **03.03.1993**
(21) Application number: 88902882.5
(22) Date of filing: 28.03.1988
(51) Int. Cl.: C05F 15/00, C12P 5/02, A01N 65/00

(54) **PROCESS FOR THE PRODUCTION OF FERTILISER**
VERFAHREN ZUR HERSTELLUNG VON DÜNGEMITTEL
PROCEDE DE PRODUCTION D'UN FERTILISANT

(43) Date of publication of application: 23.01.1991
(73) Proprietor: CLEARFIELD N.V., Curaçao (AN)
(72) Inventor: WEST, Roger, Bear Wood Bournemouth BH11 9SP (GB)
(74) Representative: Bubb, Antony John Allen
(86) International application number: GB8800234
(87) International publication number: WO8909199

(56) References cited:
- EP-A- 0 152 685
- EP-A- 0 167 984
- AU-B- 0 480 689
- DE-A- 2 354 270
- FR-A- 1 146 820
- FR-A- 2 265 288
- FR-A- 2 398 110
- FR-A- 2 516 542
- Equinoxe, no. 3, June/July 1985, (Nantes,FR), P. Manclière:"Méthanisation des algues:De l'énergie à revendre ?", pages 7-12, see pages 10-11
- Progress in Energy and Combustion Science, volume 8, no. 2, 1982, Pergamon Press Ltd, (Oxford,GB),P.N. Hobson et al.:"Production and use of biogas in agriculture", pages 135-158, see page 152, left-hand column, lines 10-30

## Description

The present invention relates to a process for the production of fertiliser and more especially to a method of producing a concentrated stable liquid fertiliser by the anaerobic microbial conversion of a mixture of livestock manure and seaweed.

Both seaweed and animal manures are recognised for their fertiliser value in the cultivation of plants. Both contain the principal plant nutrients nitrogen, phosphorus and potassium plus many minor elements (e.g. Magnesium, sulphur, sodium, calcium, zinc, iron, copper and manganese). In addition, seaweed is known to contain a range of chemical substances which are collectively known as plant growth hormones (e.g. cytokinins, gibberellins and betaines).

It is known that a liquid fertiliser can be produced from seaweed by autoclaving seaweed meal at a temperature above 100°C and at superatmospheric pressure in an aqueous solution containing sodium or potassium bicarbonate, see G.B. 664,989. Although a solution containing most of the nutrients can be obtained by filtration it is not believed to contain the more sensitive plant hormones. It is considered that such hormones would be reduced at such high processing temperatures and pressures. A further disadvantage of this process is that chemicals have to be used to prevent the alginate in the seaweed causing the extracted concentrate to set like a jelly.

It has further been proposed - see G.B. 1,599,760 to obtain a liquid product useful as a fertiliser by the anaerobic digestion of organic matter fed in slurry form to an anaerobic converter containing a culture of micro-organisms derived from a suitable source. Although a variety of sources of organic matter are suggested, including seaweed residues and pig slurry, the liquid products of this process are indicated as being of value to plant growth only in so far as the appropriate inorganic chemicals are contained in the resulting solution.

It is an object of the present invention to provide an improved process for the extraction of plant growth nutrients from seaweed in liquid form which yields not only the inorganic chemicals but also conserves the more sensitive plant growth hormones that would be destroyed by high processing temperatures. It is a further object of the invention to provide such a process that requires a low energy input.

In accordance with the present invention, it has been found that by feeding an anaerobic digester with a starting product as a slurry containing seaweed in a form that contains the desired plant growth hormones and animal manure, with a relatively low ratio between the respective solids contents of the seaweed and animal manure, the resulting digestion process is effective to solubilise not only the inorganic chemicals useful to plant growth but also the plant growth hormones of the seaweed.

The ratio of solids contents of the seaweed and animal manure may range, for example, between 1/3 and 3/1 animal manure/seaweed, with the solids content of the feed mixture preferably being in the region of 10-20%.

The process of the invention thus involves the use of an anaerobic digester, which is a closed, thermally insulated vessel fitted with facilities for heating and mixing the contents and outlets for digested slurry and biogas (methane and carbon dioxide) which evolves during the fermentation process. Although the digestion can be operated under a batch regime, it is preferable to run the system semi-continuously, feeding and discharging small volumes of slurry several times a day to achieve the required volumetric retention time. The retention time is determined according to the type of feed materials used but is typically in the range 10 to 20 days. Digester operating temperature will be preferably in the mesophilic range, typically around 35°C.

Preparation of the digester feed material involves mixing seaweed, preferably in the form of seaweed meal, and livestock manure with, if necessary, additional water to form a slurry. The optimum ratio of seaweed to manure, based on dry solids analyses, is dependent upon the particular raw materials used and the final product analysis required. The important factor is that the feed composition must be such as to promote the maintenance of healthy microbial populations. For example, the optimum ratio of seaweed to pig manure solds has been found to be around 1:1, with the feed dry solids analysis as high as 13%. There are usually inhibition problems with trying to digest animal slurries at high solids levels due to high ammonia levels. By mixing with seaweed, which is about 45% carbohydrate, a better carbon:nitrogen ratio has been found to be achieved, which promotes good digestion. The upper limits, in digestion terms, for solids in the present mixture are more likely to be set by the physical problems of pumping and mixing very thick slurries. Product analyses were found to be independent of retention times in the range 10 to 20 days, thereby allowing great flexibility in the rate of production from the digester installation.

The product of the process may be applied by sprayer as a foliar feed, in which case it is necessary to remove solid particles which would cause blockages. Agricultural sprayers may typically have gauze filters as fine as 150µm. Solids removal can be achieved by use of a suitable filtration device such as a vacuum assisted filter belt press, designed to squeeze the liquid through a woven belt with sub 150µm apertures. The dewatered solids can be used as the basis for a soil conditioner byproduct.

To reduce the volume of the liquid product for the purposes of economic packaging and transport and convenience of use, it is advantageous to have a concentration stage. In this the water is removed without the use of high temperatures. This requirement can be met by the use of the principle of membrane separation in the form of reverse osmosis. Preferably the filtrate supplied to the reverse osmosis plant is acidified from its natural pH in the typical range 7.8 - 8.3 to below pH7, for example using orthophosphoric acid or acetic acid. Strongly oxidising acids should be avoided as they are likely to denature the more sensitive growth hormones in the product. The resultant concentrate has a typical dry solids analysis of 15%, with which soluble additives may be blended to meet particular product requirements.

Stabilisation of the product can be achieved in two ways. Firstly, acidification of the concentrate, preferably to below pH 6.5, inhibits further microbial acitivity and keeps ammoniacal nitrogen in solution. Secondly, the addition of formaldehyde solution at the rate of up to 2% by volume to the concentrated product acts as a biocide and odour modifier.

### EXAMPLE

In one example of the process of the invention, the process raw materials were pig slurry with a dry solids concentration of 10%, i.e. dung and urine collected beneath slats in an intensive meal-fed pig fattening unit, and commercial Ascophyllum seaweed, in the form of meal with a dried solids content of 89%. Such seaweed meal is commercially available in the form of dried, ground seaweed and is conventionally used for the production of alginate. The blended feed slurry comprised 50% pig solids and 50% seaweed solids with a mixture dry solids concentration of approximately 13%. This was achieved by calculating the solids present in the given volume of pig slurry and adding seaweed meal to give the equivalent dry solids.

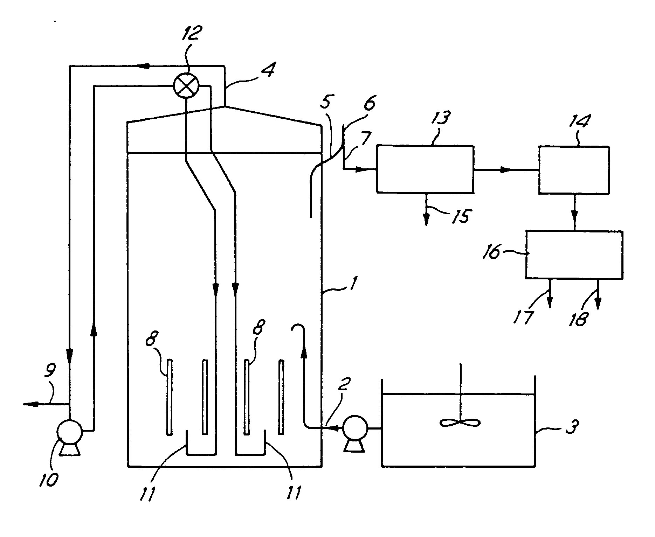

The process plant utilised in this example of the invention is illustrated diagrammatically in the accompanying drawing which is a sectional elevation of an anaerobic digester, with associated equipment shown in block form.

The anaerobic digester comprises a closed tower 1 provided in the region of the lower end with an inlet 2 through which the starting materials can be pumped from a preliminary mixing tank 3. At the upper end the tower is provided with an outlet 4 for biogas, and a weir 5 constructed in the form of a branched outlet pipe having an upwardly directed vent outlet 6 and a downwardly directed outlet 7 for the product of the digester. The lower region of the tower 1 is provided with an plurality of heat exchangers 8, typically four in number, constructed in the form of hollow tubular jackets through which heating medium can be recirculated via pipe work not shown and connected, for example, to a heating system fired by the biogas product from the digester. The outlet 4 of the digester is coupled on the one hand to an outlet 9 connected to a gas storage system, and on the other hand to an inlet of a recirculating pump 10 by means of which the biogas can be recirculated through injectors 11 directed upwardly into the bases of the vertical hollow heat exchangers 8. A distributor valve 12 may serve to connect each injector 11 in turn to the outlet of the pump 10, so that jets of the biogas are forced through each of the heat exchangers 8 at regular intervals. Efficient dispersal of the solid content of the starting product into the contents of the digester can thus be achieved whilst promoting circulation thereof through the heat exchangers.

An anaerobic digester was operated under steady state conditions in a semi-continuous mode, the heat exchangers being controlled to maintain a temperature of 37°C, and the rate of feed of starting product from the mixing tank 3 being such as to achieve hydraulic retention times of 10, 15 and 20 days, depending upon production requirements. The digester was fed hourly during the day, with simultaneous mixing in the manner described above to disperse the feed into the digester contents.

An important benefit of digestion was found to be that the alginate (constituting part of the carbohydrate above) from the seaweed is broken down so that the extract concentrate does not set like a jelly. The fact that alginate is denatured during digestion and that a large proportion of the biogas generated in the digestion process is apparently due to the presence of the seaweed is a surprising result which is contrary to the indications of prior research that the long chain molecules in alginate were resistant to attack by anaerobic bacteria.

Digested slurry with a dry solids content of 8.5% was displaced over the digester overflow weir 5 to a vacuum assisted belt press 13, fitted with a woven textile belt with approximately 125µm apertures. The resultant filtered liquid of 4.7% dry solids content was stored in a holding tank 14. Dewatered fibre of 24% dry solids content was also produced as indicated by outlet arrow 15.

Concentration of the filtered liquid was achieved by firstly acidifying with orthophosphoric acid to reduce the pH from 7.8 to 6.0, then recirculating through a reverse osmosis unit 16, operating at 40 bar pressure and a temperature of 35°C, until a product solids concentration of 15% was reached, as indicated diagrammatically by outlet arrow 17, waste water being discharged at 18.

Product stabilisation and odour modification was achieved by adding 1% v/v formaldehyde solution prior to storage.

## Claims

1. A process for extracting plant growth hormones from seaweed, characterised in that the seaweed is fed to an anaerobic digester in the form of a slurry in admixture with animal manure whereby such hormones are obtained in the liquid phase of the product of the digester.

2. A process according to Claim 1 wherein said slurry of seaweed and animal manure is prepared utilising seaweed in the form of seaweed meal.

3. A process according to Claim 1 or 2 wherein said animal manure is pig slurry.

4. A process according to any one of Claims 1-3, wherein the slurry of seaweed and animal manure comprises a ratio of seaweed/animal manure of from 1:3 to 3:1 solids content, by weight.

5. A process according to Claim 4, wherein said ratio is approximately 1:1.

6. A process according to any one of Claims 1-5, wherein the said digester is operated to provide a hydraulic retention time of the contents of from 10 to 20 days, and the content of the digester is maintained at a temperature in the mesophilic range.

7. A process according to Claim 6, wherein said temperature is approximately 35°C.

8. A process according to any one of Claims 1-7, wherein the initial solids content of said slurry of seaweed/animal manure is from 10-15%, by weight.

9. A process according to any one of Claims 1-8, wherein the solids content of the slurry within the anaerobic digester is mantained in suspension therein by forced recirculation of the biogas product of the digester through injector nozzles located in the lower region of the digester.

10. A liquid organic foliar feed produced by filtration and concentration of the product of the digestion process according to any one of Claims 1-9.

## Patentansprüche

1. Verfahren zum Extrahieren von Pflanzenwachstumshormonen aus Meerespflanzen, dadurch gekennzeichnet, daß die Meerespflanzen einem anaeroben Digester in Form eines dünnflüssigen Breies in Beimischung mit Tierdung zugeführt werden, wobei derartige Hormone in der flüssigen Phase des Produktes des Digesters erhalten werden.

2. Verfahren nach Anspruch 1, wobei der dünnflüssige Brei aus Meerespflanzen und Tierdung durch Verwendung von Meerespflanzen in Form von Meerespflanzenmehl hergestellt ist.

3. Verfahren nach Anspruch 1 oder 2, wobei der Tierdung Schweineschlamm ist.

4. Verfahren nach einem der Ansprüche 1 bis 3, wobei der dünnflüssige Brei aus Meerespflanzen und Tierdung aus einem Verhältnis Meerespflanzen/Tierdung von 1:3 bis 3:1 Festsubstanzgehalt nach Gewicht besteht.

5. Verfahren nach Anspruch 4, wobei das Verhältnis ungefähr 1:1 ist.

6. Verfahren nach einem der Ansprüche 1 bis 5, wobei der Digester betrieben wird, um für eine hydraulische Bewahrungszeit des Inhaltes von 10 bis 20 Tagen zu sorgen, und der Inhalt des Digesters auf einer Temperatur innerhalb des mesophilischen Bereiches gehalten wird.

7. Verfahren nach Anspruch 6, wobei die Temperatur ungefähr 35°C beträgt.

8. Verfahren nach einem der Ansprüche 1 bis 7, wobei der anfängliche Festsubstanzgehalt des dünnflüssigen Breies aus Meerespflanzen/Tierdung 10 bis 15 % nach Gewicht beträgt.

9. Verfahren nach einem der Ansprüche 1 bis 8, wobei der Festsubstanzgehalt des dünnflüssigen Breies innerhalb des anaeroben Digesters schwebend darin bewahrt wird durch erzwungene Rezirkulation des Biogasproduktes des Digesters durch Injektordüsen, die in dem unteren Bereich des Digesters lokalisiert sind.

10. Eine flüssige organische Blattnahrung, hergestellt durch Filtration und Konzentrierung des Produktes des Digestionsverfahrens nach einem der Ansprüche 1 bis 9.

## Revendications

1. Procédé pour extraire des hormones de croissance de plantes d'algues marines, caractérisé en ce que les algues marines sont amenées à un digesteur anaérobie sous la forme d'une boue en mélange avec du fumier animal, d'où il résulte que les hormones sont obtenues dans la phase liquide du produit du digesteur.

2. Procédé selon la revendication 1, dans lequel la boue d'algues marines et de fumier animal est préparée en utilisant les algues sous la forme de farine d'algues.

3. Procédé selon la revendication 1 ou la revendication 2, dans lequel le fumier animal est du lisier de porc.

4. Procédé selon l'une des revendications 1 à 3, dans lequel la boue d'algues marines et de fumier animal a un rapport des algues au fumier animal compris entre 1:3 et 3:1 de teneur en solides, en poids.

5. Procédé selon la revendication 4, dans lequel ledit rapport est environ 1:1.

6. Procédé selon l'une des revendications 1 à 5, dans lequel le digesteur fonctionne pour procurer un temps de séjour hydraulique du contenu allant de 10 à 20 jours, le contenu du digesteur étant maintenu à la température d'une plage mésophile.

7. Procédé selon la revendication 6, dans lequel la température est d'environ 35°C.

8. Procédé selon l'une des revendications 1 à 7, dans lequel la teneur en solides initiale de la boue d'algues marines et de fumier animal est comprise entre 10 et 15% en poids.

9. Procédé selon l'une des revendications 1 à 8, dans lequel les solides contenus dans la boue à l'intérieur du digesteur anaérobie sont maintenus en suspension par recyclage forcé du biogaz produit dans le digesteur à travers des buses d'injecteurs situés dans la région inférieure du digesteur.

10. Produit d'alimentation foliaire organique liquide obtenu par filtration et concentration du produit du procédé de digestion selon l'une des revendications 1 à 9.
